# EUROPEAN PATENT APPLICATION

(11) **EP 4 474 396 A1**
(43) Date of publication of application: **11.12.2024**
(21) Application number: 22921378.0
(22) Date of filing: 06.06.2022
(51) Int. Cl.: C07K 19/00, C07K 14/52, C12N 15/09, A61K 39/39, A61P 31/12, A61P 37/04

(54) **USE OF CCL11**

(30) Priority: 24.01.2022 CN 202210076816
(71) Applicant: Newish Technology (Beijing) Co., Ltd., Economic & Technical Development Zone Beijing 100176 (CN)
(72) Inventor: QI, Hailong, Beijing 100176 (CN); SUN, Zhongjie, Beijing 100176 (CN); JIANG, Jiandong, Beijing 100176 (CN); LI, Yuhuan, Beijing 100176 (CN); YAO, Yanling, Beijing 100176 (CN); WANG, Xudong, Beijing 100176 (CN); LIU, Defang, Beijing 100176 (CN); WANG, Xiaofang, Beijing 100176 (CN)
(74) Representative: Studio Torta S.p.A.
(86) International application number: PCT/CN2022/097092
(87) International publication number: WO 2023/137947

(57) **Abstract**

The present invention relates to the technical field of vaccine preparation, and in particular to an immune-enhancing delivery system formed by targeted antigen delivery by CCL11. The system further enhances immunogenicity by fusing a chemokine CCL11 with a corresponding antigen molecule, and adding a T2 label at a terminal of the antigen molecule. The system can be a nucleic acid vector or a fusion protein or the like to be applied to prevention and/or treatment of diseases caused by a corresponding antigen. According to the present invention, by utilizing a chemotactic binding capacity of CCL11 with a surface receptor of an immune cell such as a DC, different antigen proteins are transported to the surface of the DC, so that the efficiency of phagocytosis, processing and presentation of the DC on various antigen proteins is improved, and the effect of preventing and treating related diseases is improved. The key point of the present invention is that a T2 sequence added to an antigen can enhance an immune response.

## Description

This application claims the priority of Chinese Patent Application No. 202210076816.7, filed with the China National Intellectual Property Administration on January 24, 2022, and titled with "USE OF CCL5", which is hereby incorporated by reference in its entirety.

### FIELD

The present disclosure relates to the technical field of vaccine preparation, and in particular to an immune enhancement delivery system with targeted delivery of antigen through CCL11.

### BACKGROUND

The human body is always facing the attack of external pathogenic microorganisms and carcinogenesis of its own normal cells. The immune system is a powerful weapon of human body against pathogenic microorganisms as well as its own tumor cells. The immune system response has a process including three stages of recognition, response and effect. Each stage is completed by the coordination of the proteins, cells, tissues and organs that constitute the immune system of human body. During the recognition stage of the overall response process, the immune system needs to distinguish between self-antigens and pathogens to avoid an attack on the body. The recognition stage is followed by the response stage against specific pathogens, which includes the production of a large number of proteins and specific cells. These proteins and cells eliminate pathogens and form immunological memory against corresponding pathogens in the effect stage.

Pathogenic microorganisms such as viruses, bacteria, fungi, and parasites often cause large-scale epidemic diseases. For example, SARS-CoV-2, which broke out at the end of 2019, has spread globally. After pathogenic microorganisms invade the human body, some induce transient diseases without long-term incubation in the human body, but some pathogens can incubate in human cells and tissues for a long time and cause corresponding diseases. Therefore, prophylactic vaccines are required to prevent the spread of pathogenic microorganisms, and therapeutic vaccines are required to treat and eliminate the cells that are host to pathogenic microorganisms. This requires the immune system to generate humoral as well as cellular immune response, which is realized by the measure of injecting the human body with specific antigens of pathogenic microorganisms to cause immune recognition. Besides, it can be known from the past experience that the stronger the immune response, the better the prevention and treatment effect of the vaccine.

CCL11, as a member of the CC family of chemokines, is mainly expressed in small intestine, heart, kidneys and pancreas. Pro-inflammatory cytokines such as TNFa and IL-4 can upregulate the expression of CCL11. CCL11 plays a chemotactic function mainly through its receptor CCR3. Studies have shown that CCR3 is expressed on DCs and eosinophils. DCs are professional antigen-presenting cells, and mainly mediate antigen recognition. Eosinophils, in addition to the classic antibacterial effect, can also mediate anti-tumor immune responses by enhancing specific T cell immune responses. Based on the above, the fusion of antigen molecules and CCL11 will enhance the immunogenicity of antigen, which initiates a stronger immune response and provides options for the preparation of more effective vaccines.

Dendritic cells (DCs) are professional antigen-presenting cells and an important component of the immune system. They circulate in the blood and reside throughout the body, where they are primarily responsible for phagocytosis, processing and presentation of antigens, and play a crucial role in the immune recognition stage. The recognition degree of antigens by DCs determines the strength of the immune response.

Tumors originate from autologous tissue cells and are thus difficult to be recognized by the immune system. Compared with normal tissue cells, however, tumor cells usually have certain highly expressed or mutated oncogenes for the proliferation and survival of tumor cells. These highly expressed or mutated gene products usually have only weak immunogenicity, which will not cause a strong immune response to eliminate the tumor cells. Therefore, how to mobilize the immune system to generate a strong immune response has become the key to eliminating tumors.

Tumor vaccines induce the effector T cells in patients to exert functions by potentiating the existing anti-tumor response or activating naive T cells. Antigen-specific CD8+ cytotoxic T lymphocytes (CTL) play an important role in the anti-tumor process. DCs are the only professional antigen-presenting cells that can activate naive CD8+ T cells. They uptake, process and cross-present extracellular tumor antigens through MHC-I, and are crucial for the production of effective CTLs. Therefore, delivery of tumor antigens to DCs by conjugating DC cell surface molecules is an effective tumor therapy strategy for inducing CD8+ T cell immune responses.

At present, the proven molecules that can enhance the presentation of DCs include XCL-1, but it is still urgent to find out more DC cell surface molecules that can promote the presentation.

### SUMMARY

In view of this, the technical problem to be solved by the present disclosure is to provide an immune enhancement delivery system formed by a T2 fragment and/or CCL11 targeting delivering antigen.

The present disclosure provides:
Use of at least one of I)-VI) in improving antigen presentation effect;
I) a T2 fragment with an amino acid sequence set forth in SEQ ID NO: 4;
II) chemokine CCL11;
III) a fragment being more than 80% homologous with and functionally identical or similar to I) or II);
IV) a nucleic acid molecule encoding I) or II);
V) a nucleic acid molecule that is derived from the nucleotide sequence of the nucleic acid molecule in IV) by deletion, addition or substitution of one or more nucleotides and encode a protein functionally identical or similar to the nucleic acid molecule in IV);
VI) a nucleic acid molecule fully or partially complementary to V).

In the present disclosure, the T2 fragment consists of 31 amino acids, and has a sequence set forth in SEQ ID NO: 4. Studies have shown that the artificially modified T2 fragment has the effect of enhancing immunity in the human body.

In the present disclosure, the CCL11 is a chemokine CCL11 derived from humans or other animals, and is a full-length sequence or a fragment with CCL11 activity. In the present disclosure, it is found that CCL11 can deliver antigens to DCs, thereby improving the presentation effect and enhancing immune response.

The present disclosure also provides a fusion protein comprising CCL11 and an antigen or comprising CCL11, an antigen and a T2 fragment.

In an embodiment of the present disclosure, the fusion protein, from N-terminal to C-terminal, sequentially comprises an IgE signal peptide, CCL11, a linker, the antigen and the T2 fragment.

In the present disclosure, the antigen is derived from a virus, pathogenic microbe and/or tumor.

In the present disclosure, the CCL11 is a humanized CCL11 sequence, and the antigen is E6 protein of HPV16 and/or E7 protein of HPV16.

In some embodiments, the fusion protein, from N-terminal to C-terminal, sequentially comprises an IgE signal peptide, CCL11, a linker, E6 protein of HPV16, E7 protein of HPV16, and the T2 fragment.

In some specific embodiments, the IgE signal peptide has an amino acid sequence set forth in SEQ ID NO: 5;
the CCL11 has an amino acid sequence set forth in SEQ ID NO: 3;
the linker is (GsS)n, wherein n is 1-10; in the embodiments of the present disclosure, the linker has a sequence of GGGGGSGGGGG.

The E6 protein of HPV16 has an amino acid sequence set forth in SEQ ID NO: 1.

The E7 protein of HPV16 has an amino acid sequence set forth in SEQ ID NO: 2.

The T2 fragment has an amino acid sequence set forth in SEQ ID NO: 4.

In some specific embodiments, the fusion protein further comprises a FLAG tag sequence at the C-terminal with an amino acid sequence of DYKDDDDK, which is only a tag for identifying protein expression and does not affect the immune effect of the sequence.

The present disclosure further provides a nucleic acid encoding the fusion protein.

The nucleic acid encoding the fusion protein of the present disclosure has a nucleotide sequence set forth in SEQ ID NO: 9.

The present disclosure provides a nucleic acid fragment, which comprises the nucleic acid encoding the fusion protein of the present disclosure, 5'-UTR, 3'-UTR and 3'-end polyA, wherein the 5'-UTR is 0-globin-2, the 3'-UTR is 20-globin, and the 3'-end polyA has a length of 120 bp. The nucleic acid fragment has a structure of 5'UTR-CCL11-E6E7-3'UTR-A(120).

The present disclosure also provides a transcription unit encoding the fusion protein.

The transcription unit comprises a promoter and the nucleic acid encoding the fusion protein.

In some embodiments, the transcription unit further comprises a terminator.

In some specific embodiments, the promoter is a CMV or CMV/R promoter.

The present disclosure further provides an expression vector, comprising a vector backbone and the nucleic acid encoding the fusion protein.

In the present disclosure, the vector backbone is selected from the group consisting of pVAX1 series vectors and pVR series vectors.

The present disclosure further provides a recombinant host transformed or transfected with the expression vector.

In the present disclosure, the host cells of the recombinant host are bacteria or mammalian cells.

The present disclosure provides a method for producing the fusion protein, comprising culturing the recombinant host of the present disclosure and collecting a culture containing the fusion protein.

The present disclosure provides a delivery system for delivering antigenic substances such as viruses, bacteria, fungi and tumors to CCR3-positive antigen-presenting cells. In the system, the antigen molecule is fused with chemokine CCL11, and a T2 tag is added at the end of the antigen molecule to further enhance the immunogenicity. The system may be in the form of a nucleic acid vector or fusion protein for the prevention or treatment of diseases caused by the antigen.

The present disclosure provides use of an antigen delivery system in the manufacture of a preventive or therapeutic vaccine, wherein the delivery system comprises a ligand CCL11 to bind to CCR3.

The present disclosure provides use of the fusion protein, nucleic acid, expression vector, host, fusion protein prepared by the method, and/or the culture containing the fusion protein prepared by the method of the present disclosure in the manufacture of a product for preventing and/or treating a disease.

The present disclosure further provides a product for preventing and/or treating a disease, comprising the fusion protein, nucleic acid, expression vector, host, fusion protein prepared by the method, and/or the culture containing the fusion protein prepared by the method.

The present disclosure also provides a method for preventing and/or treating a disease, comprising administering the product for preventing and/or treating a disease of the present disclosure.

In the present disclosure, the preventing and/or treating specifically includes increasing antibody level in serum, preventing tumor formation, inhibiting tumor growth, and improving immune response ability of the body against tumors.

In the present disclosure, the disease includes diseases caused by viruses and/or pathogenic microbes, or the disease is a tumor.

In the present disclosure, the product for preventing and/or treating a disease includes a medicine and/or a vaccine. In the present disclosure, the vaccine is selected from the group consisting of a DNA vaccine, a recombinant protein vaccine and an mRNA vaccine.

In the present disclosure, the administering includes oral administration, injection and/or electroporation.

Relevant studies and multiple experiments have shown that not all CC family chemokine members fused with antigen molecules can enhance immunogenicity. Only some are used to be fused with antigen molecules to enhance the immunogenicity of antigens, initiate a stronger immune response, and provide options for more potent vaccine preparations. For example, relevant studies have indicated that the fusion of 4-1BBL-S, 4-1BBL-Fc or CD80-Fc with antigen molecules can greatly enhance the immunogenicity of antigens, but the fusion of GM-CSF, mlL-23, or IL-15SAG1 with antigen molecules does not have the effect of enhancing immunogenicity.

In the present disclosure, the chemotactic binding ability of CCL11 to surface receptors on immune cells such as DCs is utilized to transport and cross-present various antigenic proteins to the surface of DCs, which improves the efficiency of phagocytosis, processing and presentation of various antigenic proteins by DCs, and improves the effect of preventing and/or treating related diseases. The T2 fragment of the present disclosure has been determined to have an extremely strong immune enhancement effect, and can further stimulate humoral and cellular immune responses in the process of promoting antigen presentation, finally achieving the effect of inhibiting the growth of related tumors.

### BRIEF DESCRIPTION OF DRAWINGS

In order to more clearly illustrate the specific embodiments of the present disclosure or the technical solutions in the prior art, the following will briefly introduce the drawings used in the description of the specific embodiments or the prior art. Apparently, the drawings in the following description are some embodiments of the present disclosure. For those of ordinary skill in the art, other drawings can also be obtained according to these drawings without making creative effort:
FIG. 1 shows the analysis of the ability of CCL11 to chemoattract DCs and eosinophils.
FIG. 2 shows the vector maps of nucleotides encoding the fusion proteins; wherein A shows the plasmid map of pVR-CCL11-E6E7-T2, B shows the plasmid map of pVR-CCL11-E6E7, and C shows the plasmid map of pVR-E6E7.
FIG. 3 shows the expression of the target genes of the three plasmids pVR-CCL11-E6E7-T2, pVR-CCL11-E6E7 and pVR-E6E7, which is obtained by detecting the expression of the nucleotide encoding the fusion protein with a Flag tag at the C-terminal using Western blot.
FIG. 4 shows the timeline of the prophylactic immunization performed on mice with different fusion genes for later testing of specific T cell responses.
FIG. 5 shows the results of flow cytometric detection of specific T cell responses after immunization of mice with different fusion genes.
FIG. 6 shows the results of detection of specific antibody responses after immunization of mice with different fusion genes.
FIG. 7 shows the timeline of tumor inoculation in mice with different fusion genes, mRNA and protein vaccines for therapeutic immunization.
FIG. 8 shows the volume of cell tumors treated by each group.

### DETAILED DESCRIPTION

The present disclosure provides an immune enhancement delivery system with targeted delivery of antigen through CCL11. Those skilled in the art can learn from the content herein and appropriately improve the process parameters for realization. It should be particularly pointed out that all similar replacements and modifications are apparent to those skilled in the art, and they are all considered to be included in the present disclosure. The method and use of the present disclosure have been described through preferred embodiments, and it is apparent that relevant persons can make changes or appropriate modifications and combinations of the methods and use herein without departing from the content, spirit and scope of the present disclosure to implement and apply the present disclosure.

Unless otherwise defined, all technical and scientific terms used herein have the same meaning as commonly understood by those of ordinary skill in the art. For definitions and terms in the art, those skilled can refer to Current Protocols in Molecular Biology (Ausubel). The standard three- and/or one-letter code used for expressing one of 20 common L-amino acids in the art is adopted as the abbreviation of an amino acid residue.

The CCL11 of the present disclosure is an important chemokine in the human body, belonging to the CC chemokine family, and can be specifically expressed at a high level in human heart and kidneys. The present disclosure shows that CCL11 can be used to deliver substances to professional antigen-presenting cells, especially DCs and eosinophils, so as to enhance the presentation effect of DCs and the recruitment effect of T cells.

In the present disclosure, the CCL11 can be a humanized fragment, or fragments from other animals, such as murine, rabbit's, simian and porcine fragments. It can be a complete CCL11, or a fragment or mutant with CCL11 activity, which is not limited by the present application. In the examples of the present application, humanized CCL11 was used as the experimental object to demonstrate the improvement of the antigen presentation effect of CCL11. The humanized CCL11 has an amino acid sequence set forth in SEQ ID NO: 3.

In the fusion protein of the present disclosure, the T2 fragment is modified from a short peptide at the C-terminal of bacteriophage T4 fibrin, and is an exogenous sequence in terms of species source. This sequence is completely absent in the human body, and will not cause problems of killing other human proteins after immune enhancement. It has been reported that this sequence can promote the trimerization of certain proteins under certain conditions. In the present disclosure, it is found that the T2 fragment, as an artificially modified polypeptide sequence, has an immune enhancing effect in the human body. In the present disclosure, the T2 fragment consists of 31 amino acids, and has a sequence set forth in SEQ ID NO: 4.

The fusion protein of the present disclosure comprises at least one antigen. For example, it may comprise 1, 2, 3, 4, 5, 6, 7, 8, 9, 10 or more antigens. In the present disclosure, experiments were conducted on the fusion effect of one or two antigens, which all showed good results.

The antigen of the present disclosure is derived from proteins derived from viruses, bacteria, fungi, parasites and/or tumors. In some embodiments, the antigen is derived from capsid proteins or non-structural proteins of viruses, membrane proteins or flagellin of pathogenic microbes, or surface antigens of tumors. It may be a complete fragment or an antigenic determinant thereof. It may contain only one antigenic determinant, or may be composed of multiple antigenic determinants in series, or may contain two or more repeats in series of one antigenic determinant.

In the present disclosure, the virus is selected from the group consisting of HPV, HCMV, EBV, HCV, HIV, HBV, VZV, a coronavirus, and a combination thereof.

In the present disclosure, the tumor is selected from the group consisting of liver cancer, cervical cancer, ovarian cancer, lung cancer, head and neck cancer, prostate cancer, breast cancer, blood cancer, melanoma, nasopharyngeal carcinoma, and a combination thereof.

In some embodiments, the antigen is E2, E5, E6 and/or E7 protein of HPV or a mutant epitope thereof. The HPV includes viruses of various HPV subtypes, for example, HPV6, HPV11, HPV16, HPV18, HPV31, HPV33, HPV35, HPV39, HPV45, HPV51, HPV52, HPV56 and/or HPV58.

In some embodiments, the antigen is LMP1, LMP2, EBNA1 of EB virus or a mutant epitope thereof.

In some embodiments, the antigen is S protein, N protein, E protein, M protein of a coronavirus or an epitope thereof. The coronavirus is SARS-CoV, MERS-CoV and/or SARS-CoV-2.

In some embodiments, the antigen is pan-oncoproteins such as VEGFR2, Survivin, and FAP.

In some embodiments, the antigen is GPC3 protein and/or AFP protein of liver cancer.

In some embodiments, the antigen is PSA, PSMA, PSCA, PAP and/or STEAP1 of prostate cancer.

In some embodiments, the antigen is a dominant epitope of Her2/neu and/or BCAR3 of breast cancer.

In some embodiments, the antigen is MAGE-A3, ISR2, NY-ESO-1, Melan A, gp100, Tyrosinase, TRP1 and/or TRP2 of melanoma.

In some embodiments, the antigen is immunoglobulin idiotype, immunoglobulin κ-chain and/or immunoglobulin λ-chain of blood cancer.

In some embodiments, the antigen is AIM2, HT001, TAF1B, Micoryx and/or TGFβRII of colorectal cancer.

In some embodiments, the antigen is folate receptor-α of ovarian cancer.

In some embodiments, the antigen is P53, IDH1/2, BAGE, GAGE1, GAGE2, CAG3, RAGE, CEA, CDK4, CASP-8, KRAS, bcr/abl and/or MUC-1 of various proto-oncogenes, antioncogenes and/or tumor-specific antigens.

In the present disclosure, the chemotactic binding ability of CCL11 to surface receptors on immune cells such as DCs is utilized to transport and cross-present the above antigenic proteins to the surface of DCs, which improves the efficiency of phagocytosis, processing and presentation of various antigenic proteins by DCs, and improves the effect of preventing and/or treating related diseases. In previous preliminary experiments, the presentation efficiency of multiple antigens of the above proteins including E6 or E7 protein of HPV16 has been demonstrated to be improved by CCL11. In the embodiments of the present disclosure, E6 and E7 proteins of HPV16 are taken as examples to prove that CCL11 can improve the efficiency of antigen protein presentation, and other proteins fused with CCL11 can also have a good effect.

In the present disclosure, in order to ensure smooth folding of each functional fragment in the fusion protein without being affected by steric hindrance, a linker is added between the fragments, wherein the linker between CCL11 and the antigenic protein of HPV is GGGGGSGGGGG. Different antigens can be linked by (G₅S)ₙ and/or AGA.

In the present disclosure, in order to improve the expression effect of the fusion protein, a signal peptide that promotes the secretion of the fusion protein to the extracellular space is added to the N-terminal of CCL11. In some embodiments, the signal peptide is an IgE signal peptide. Specifically, it has an amino acid sequence set forth in SEQ ID NO: 5.

In the present disclosure, in order to facilitate the purification of the fusion protein, a tag is added to the C-terminal of the fusion protein. The tag is selected from recombinant protein purification tags well known in the art. In some embodiments, the tag is DYKDDDDK.

In some specific embodiments, the fusion protein has a structure which, from N-terminal to C-terminal, sequentially comprises an IgE signal peptide, a humanized CCL11 protein sequence, a linker sequence (GGGGGSGGGGG), an E6 E7 protein sequence, a T2 fragment sequence, and a Flag tag sequence. Specifically, it has an amino acid sequence set forth in SEQ ID NO: 15.

In the present disclosure, the nucleic acid encoding the protein may be DNA, RNA, cDNA or PNA. In an embodiment of the present disclosure, the nucleic acid is in the form of DNA or RNA. The DNA form includes cDNA, genomic DNA or synthetic DNA. The DNA may be single-stranded or double-stranded. The nucleic acid can comprise nucleotide sequences that have different functions, for example, a coding region and a non-coding region such as a regulatory sequence (e.g., a promoter or a transcription terminator). The nucleic acid can be linear or circular in topology. The nucleic acid can be, for example, a portion or a fragment of a vector, such as an expression or cloning vector. The nucleic acid may be obtained directly from natural sources, or may be prepared with the aid of recombinant, enzymatic or chemical techniques. The RNA form is mRNA obtained by gene transcription or the like.

In the present disclosure, the DNA sequence for expressing the fusion protein is optimized, and such optimization includes but is not limited to: codon usage bias, elimination of secondary structures (such as hairpin structures) that are not conducive to expression, changes in GC content, CpG dinucleotide content, secondary structure of mRNA, cryptic splice sites, early polyadenylation sites, internal ribosome entry and binding sites, negative CpG islands, RNA instability region, repeats (direct repeats, reverse repeats, etc.) and restriction sites that may affect cloning.

The prevention of the present disclosure refers to reducing the risk of tumor occurrence by administering the drug of the present disclosure before the tumor occurrence. The treatment of the present disclosure refers to inhibiting tumor growth, reducing tumor volume or delaying tumor growth by administering the drug of the present disclosure after tumor occurrence. In the examples of the present disclosure, the mouse transplanted tumor cell TC-1 is used as the experimental object to demonstrate the effect of the fusion protein vaccine.

The present disclosure further provides a transcription unit of the fusion protein, and the transcription unit refers to the DNA sequence beginning from the promoter and ending at the terminator. Regulatory fragments may also be comprised on either side of or between the promoter and terminator, and may include promoters, enhancers, transcription termination signals, polyadenylation sequences, origins of replication, nucleic acid restriction sites, and homologous recombination sites, such as promoter enhancers and poly(A) signals, which are operably linked to the nucleic acid sequence. The transcription unit provided by the present disclosure comprises a CMV or CMV/R promoter, a CMV enhancer and a nucleic acid fragment encoding the fusion protein.

The expression vector of the present disclosure refers to a recombinant nucleic acid vector, which is a recombinant DNA molecule that comprises a desired coding sequence and suitable nucleic acid sequences necessary for the expression of an operably linked coding gene in a specific host organism. Nucleic acid sequences necessary for expression in prokaryotic cells include a promoter with an optional operator sequence, a ribosome binding site and possibly other sequences. It is known that a promoter, enhancer, terminator and polyadenylation signals are used in prokaryotic cells. Once transformed into a suitable host, the vector can replicate and function independently of the host genome, or, in some cases, it can be integrated into the genome. In this specification, "plasmid" and "vector" are sometimes used interchangeably because plasmid is currently the most commonly used form of vector. However, the present disclosure is intended to include such other forms of expression vectors, which serve equivalent functions, and are or will become known in the art, including but not limited to: plasmids, phage particles, viral vectors and/or potential genomic insertions. In a specific embodiment, the nucleic acid encoding the fusion protein provided by the present disclosure can be constructed in various eukaryotic expression vectors. For example, the vector backbone can be a pVAX1 series vector, or a pVR series vector (see Chinese patent ZL 202110624820.8).

The host cell of the present disclosure is a prokaryotic or eukaryotic host containing a nucleic acid vector and/or a target gene. The host cells are transformed or transfected with vectors constructed using recombinant DNA techniques. Such transformed host cells are capable of replicating the vector encoding the protein or expressing the desired protein.

In the embodiments of the present disclosure, the fusion protein is prepared by a method of inducing the expression of the recombinant host, and the obtained culture can be bacteria, cells and culture solution obtained from culture, or substances obtained by extraction and/or purification from the above cultures.

The product for preventing and/or treating a disease provided by the present disclosure comprises the fusion protein, nucleic acid, expression vector, host, the fusion protein prepared by the method, and/or the culture containing the fusion protein prepared by the method. In the present disclosure, the product for preventing and/or treating a disease includes a medicine and/or a vaccine. The vaccine further comprises a pharmaceutically acceptable carrier, an excipient and/or an adjuvant. The medicine further comprises a pharmaceutically acceptable auxiliary material.

The prevention of the present disclosure refers to reducing the risk of disease occurrence by administering the product for preventing and/or treating a disease of the present disclosure before the disease occurrence. The treatment of the present disclosure refers to improving a disease, inhibiting the development of a disease, and restoring patients to health by administering the product for preventing and/or treating a disease of the present disclosure after the disease occurrence. For example, the use of the product of the present disclosure in preventing and/or treating tumors can increase the level of antibodies in serum, inhibit tumor growth, reduce tumor volume or delay tumor growth. In the examples of the present disclosure, the mouse transplanted tumor cell TC-1 is used as the experimental object to demonstrate the effect of the fusion protein vaccine, and good effects were obtained.

In the examples of the present disclosure, the amino acid sequences of the fragments involved and the coding nucleic acid fragments are shown in Table 1:

**Table 1 Amino acid sequences and coding nucleic acid fragments involved in the present disclosure**

| | |
|---|---|
| SEQ ID NO: 1 | Amino acid sequence of E6 protein of HPV16 |
| SEQ ID NO: 2 | Amino acid sequence of E7 protein of HPV16 |
| SEQ ID NO: 3 | Amino acid sequence of CCL11 |
| SEQ ID NO: 4 | Amino acid sequence of T2 fragment |
| SEQ ID NO: 5 | IgE signal peptide |
| SEQ ID NO: 6 | Nucleotide sequence of plasmid pVR-CCL11-E6E7-T2 |
| SEQ ID NO: 7 | Nucleotide sequence of plasmid pVR-CCL11-E6E7 |
| SEQ ID NO: 8 | Nucleotide sequence of plasmid pVR-E6E7 |
| SEQ ID NO: 9 | DNA sequence encoding the fusion protein in plasmid pVR-CCL11-E6E7-T2 |
| SEQ ID NO: 10 | DNA sequence encoding the fusion protein in plasmid pVR-CCL11-E6E7 |
| SEQ ID NO: 11 | DNA sequence encoding the fusion protein in plasmid pVR-E6E7 |
| SEQ ID NO: 12 | mRNA sequence encoding the fusion protein in plasmid pVR-CCL11-E6E7-T2 |
| SEQ ID NO: 13 | mRNA sequence encoding the fusion protein in plasmid pVR-CCL11-E6E7 |
| SEQ ID NO: 14 | mRNA sequence encoding the fusion protein in plasmid pVR-E6E7 |
| SEQ ID NO: 15 | Amino acid sequence of the fusion protein in plasmid pVR-CCL11-E6E7-T2 |
| SEQ ID NO: 16 | Amino acid sequence of the fusion protein in plasmid pVR-CCL11-E6E7 |
| SEQ ID NO: 17 | Amino acid sequence of the fusion protein in plasmid pVR-E6E7 |

The embodiments of the present disclosure will be described in detail below in conjunction with examples, but those skilled in the art will understand that the following examples are only for illustrating the present disclosure, and should not be considered as limiting the scope of the present disclosure. The examples without specific conditions indicated were carried out according to the conventional conditions or the conditions recommended by the manufacturers. The reagents or instruments used without manufacturer indicated were all conventional commercially available products.

### Example 1

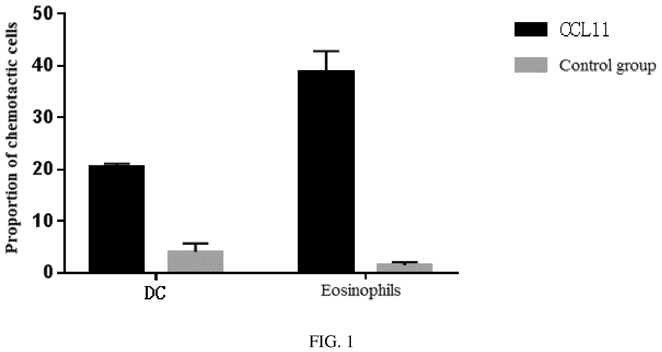

DCs and eosinophils were isolated from mouse lymph nodes and peripheral blood for chemotaxis experiments. The above isolated cells were placed in the upper chamber of a chemotaxis chamber (Transwell carbonate membrane chamber: 5 µm; Costar, Cat: 3422), and the cells were added at 1×10⁶ cells/100 µL/well based on the previous work in the laboratory. The chemotaxis chamber was put into the lower chamber containing 600 µL of medium containing chemokine CCL11. A spontaneous migration control group and a CCL11 cytokine group were set up simultaneously, and added with the same number of cells. 3 replicate wells were set up. The purified recombinant (*E.coli*) murine CCL11 protein was used as chemokine CCL11. According to the previous work in the laboratory, 100 ng/mL was the optimal dose for chemotactic efficiency. After 4 h, the cells in the lower chemotaxis chamber were collected, and the chemotactic ability of CCL11 to various immune cells was analyzed by flow cytometry. The results show that CCL11 could effectively recruit various immune cells from the upper chamber to the lower chamber (P<0.001) (FIG. 1).

### Example 2 Antigen design scheme of fusion gene or protein vaccine and construction and preparation of mammalian expression plasmid

Construction of plasmid pVR-CCL11-E6E7-T2: A fusion protein CCL11-E6E7-T2 was constructed based on E6 and E7 proteins of human papillomavirus subtype HPV16, humanized CCL11 protein and T2 fragment. An IgE signal peptide with an amino acid sequence of MDWTWILFLVAAATRVHS was connected to the N-terminal of the fusion protein CCL11-E6E7-T2, and a Flag tag consisting of 8 amino acids of DYKDDDDK was connected to the C-terminal of the fusion protein CCL11-E6E7-T2.

The finally obtained fusion protein, from N-terminal to C-terminal, sequentially comprised an IgE signal peptide, a humanized CCL11 protein sequence, a linker sequence (GGGGGSGGGGG), an E6 protein sequence, an E7 protein sequence, a T2 fragment sequence and a Flag tag sequence.

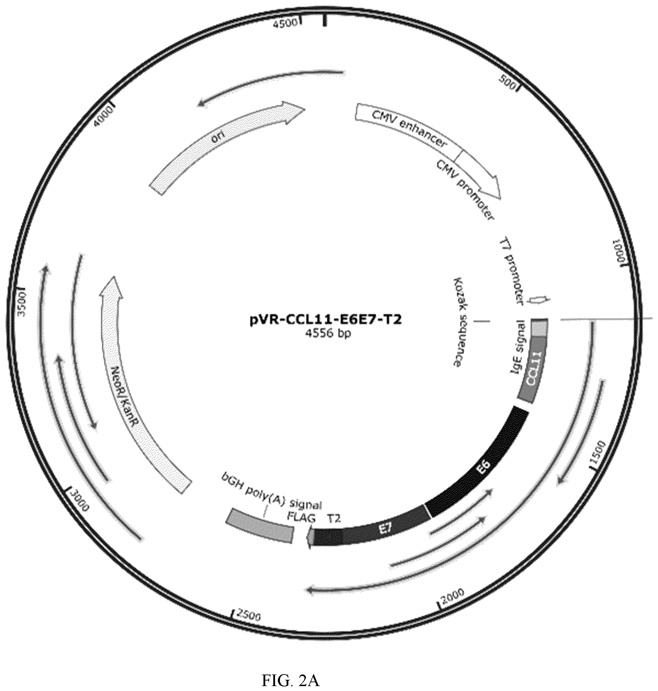

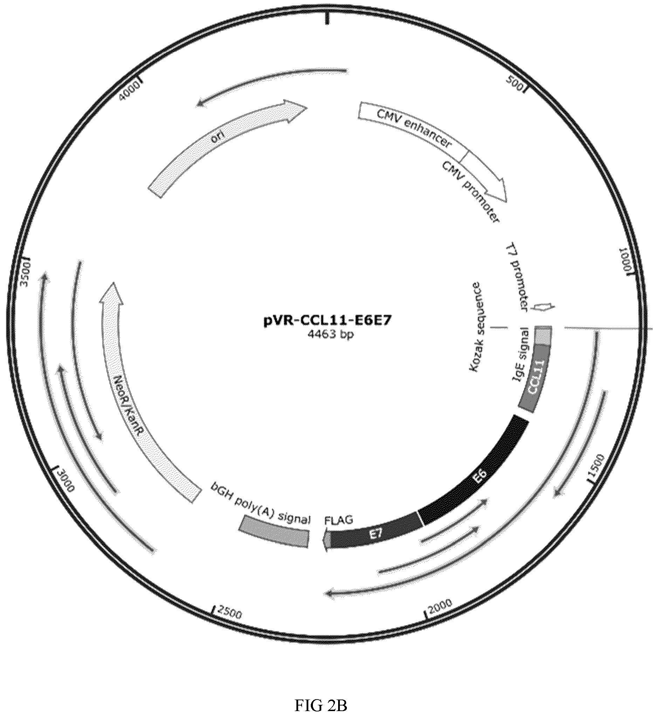

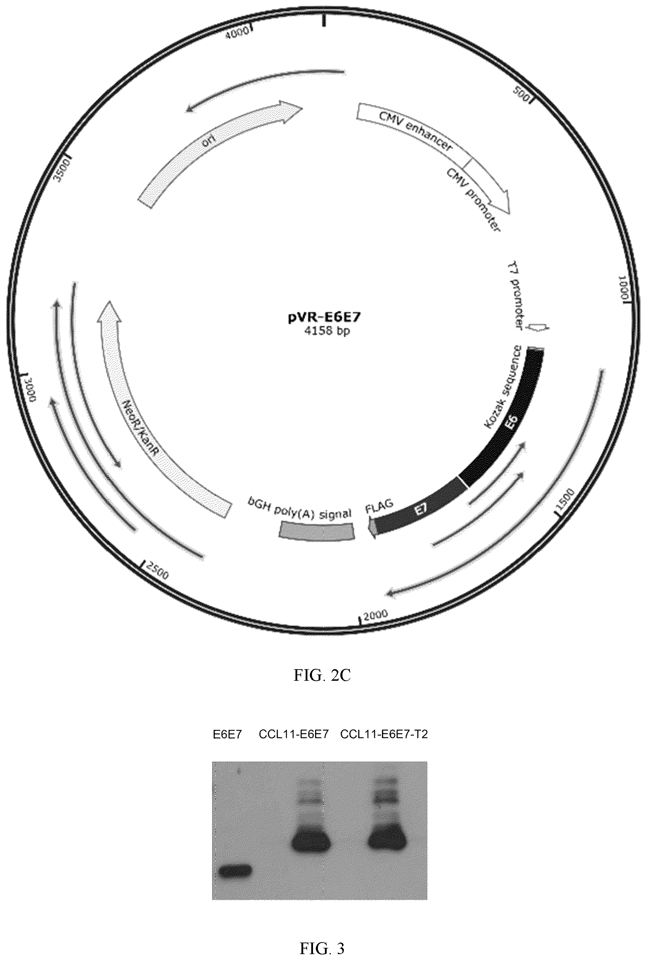

The amino acid sequence of the fusion protein was subjected to codon optimization for expression preference of mammalian cells. The gene sequence of the fusion protein, determined as SEQ ID NO: 8, was then synthesized and inserted into the corresponding polyclonal sites region of pVR plasmid vector so as to express the fusion protein in the correct codon translation sequence. The finally constructed plasmid was named plasmid pVR-CCL11-E6E7-T2, as shown in A in FIG. 2.

Construction of plasmid pVR-CCL11-E6E7: Similarly, the finally constructed fusion gene, from N-terminal to C-terminal, sequentially comprised an IgE signal peptide, a humanized CCL11 protein sequence, a linker sequence (GGGGGSGGGGG), an E6E7 protein sequence, and a Flag tag sequence.

The amino acid sequence of the fusion protein was subjected to codon optimization for expression preference of mammalian cells. The gene sequence of the fusion protein, determined as SEQ ID NO: 10, was then synthesized and inserted into the corresponding polyclonal sites region of pVR plasmid vector so as to express the fusion protein in the correct codon translation sequence. The finally constructed plasmid was named plasmid pVR-CCL11-E6E7, as shown in B in FIG. 2.

Construction of plasmid pVR-E6E7: The E6 and E7 protein sequences of human papillomavirus subtype HPV16 were preceded by an IgE signal peptide with an amino acid sequence of MDWTWILFLVAAATRVHS, and followed by a Flag tag consisting of 8 amino acids of DYKDDDDK.

The finally obtained fusion protein, from N-terminal to C-terminal, sequentially comprised an IgE signal peptide, an E6E7 protein sequence, and a Flag tag sequence, as shown in C in FIG. 2.

The amino acid sequence of the fusion protein was subjected to codon optimization for expression preference of mammalian cells. The gene sequence of the fusion protein, determined as SEQ ID NO: 11, was then synthesized and inserted into the corresponding polyclonal sites region of pVR plasmid vector so as to express the fusion protein in the correct codon translation sequence. The finally constructed plasmid was named plasmid pVR-E6E7.

Specifically, the plasmid patterns constructed in the experiments in this example are actually: pVR-CCL11-antigen-T2, pVR-CCL11-antigen and a control plasmid pVR-antigen. In the experiment of this example, the E6E7 fusion protein of HPV 16 subtype was used as the antigen.

### Example 3

*In vitro* cell transfection experiments with the constructed plasmids were performed. The vectors comprising antigens of HPV16 E6 and E7 proteins constructed in Example 2 were taken as examples, and the vectors comprising other antigens were transfected by the same method.

24 h before transfection, 1×10⁶ HEK293T cells were plated in a 6-well cell culture plate. The transfection experiment was started when the cell density reached more than 80%. Before transfection, cell culture medium and serum-free Opti-MEM medium were pre-warmed in a 37°C water bath. During transfection, 3 µg of control vector, pVR-CCL11-E6E7-T2 expression vector, pVR-CCL11-E6E7 expression vector, and pVR-E6E7 expression vector and 12 µL of PEI transfection reagent were added to 200 µL of serum-free Opti-MEM, mixed evenly, and then left to stand at room temperature for 10 min. The cells to be transfected were fed with fresh medium, gently added to the above transfection system, and shaken gently. The cells were then placed in the cell culture incubator again for 6 h of culture and then fed with fresh medium. After 48 h of transfection, the cells were collected, and the expression effect of the E6E7 fusion gene plasmid in HEK293T cells was then detected by Western Blot.

The collected cells were added with 60 µL of 0.5% NP40 lysis buffer containing PMSF or Cocktail protease inhibitor, resuspended fully and lysed by rotating at 4°C for 30 min. The lysate was centrifuged at 12,000 rpm at 4°C for 10 min. The supernatant was collected into a new 1.5 mL EP tube, and the precipitate was discarded. 5×SDS-PAGE protein loading buffer was added according to the actual volume of the sample and mixed well. Then the sample was heated in an air bath at 100°C for 10 min, subjected to Western blot immediately, and detected using Flag tag antibody (Sigma, F3165). The results are shown in FIG. 3 that the control vector had no protein expression, and the position showing the size of the expressed proteins of pVR-CCL11-E6E7-T2 and pVR-CCL11-E6E7 was significantly higher than that of pVR-E6E7, indicating that the plasmids pVR-CCL11-E6E7-T2 and pVR-CCL11-E6E7 of the experimental group and the plasmid pVR-E6E7 of the control group can all be smoothly and normally expressed in mammalian cells.

### Example 4

The effects of chemokine CCL11 and T2 fragment on cell-specific T cell responses induced by the fusion gene vaccine were explored. The vaccines comprising antigens of HPV16 E6 and E7 proteins were taken as an example and the steps for inducing T cell responses by vaccines comprising other antigens were the same.

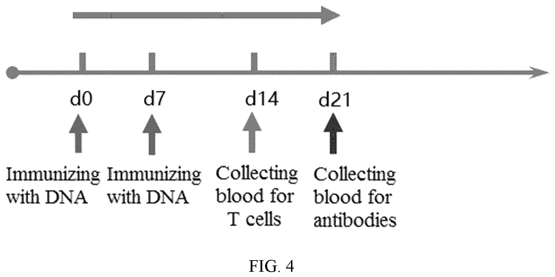

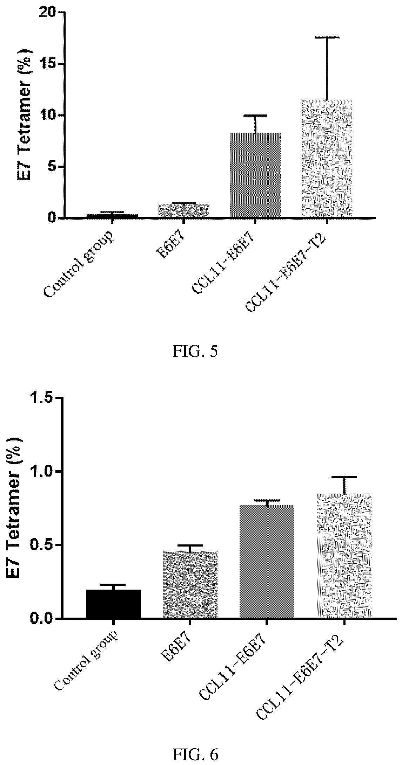

Given that the fusion gene can be normally expressed in mammalian cells, plasmids pVR-CCL11-E6E7-T2, pVR-CCL11-E6E7, and pVR-E6E7 were extracted separately, and the mice were immunized with the plasmids of 25 µg by an in vivo gene transfer instrument from TERESA. There were four groups including a negative control PBS group, 5 mice in each group. The mice were immunized according to the immunization strategy marked in the timeline in FIG. 4, and blood was collected from the mice in each group on D14 and added to the PBS solution containing heparin. All the samples were centrifuged at 3000 rpm for 5 min. The supernatant was discarded, and the remaining pellets were dispersed by shaking, and then added with 1 mL of red blood cell lysis solution for 1 min of lysis at room temperature. Then all the samples were centrifuged at 1200 rpm for 6 min. The supernatant was discarded, and the remaining pellets were washed once with 700 µL of PBS solution and centrifuged for the second time at 1200 rpm for 6 min. After the supernatant was discarded, the pellets were resuspended with 300 µL of inactivated 1640 medium containing 10% FBS, and added with 1 µL of E7-tetramer for 1 h of staining. Flow staining: CD8-FITC; E7-tetramer-PE. The flow results are shown in FIG. 5. The results show that in the comparison between groups at the same dose, the number of E7-specific T cells in CCL11-E6E7-T2 group was far more than that in the E6E7 group. In the comparison between groups at the same dose, the number of E7-specific T cells in CCL11-E6E7-T2 group was more than that in the CCL11-E6E7 group. In the comparison between groups at the same dose, the number of E7-specific T cells in CCL11-E6E7 group was more than that in the E6E7 group. This shows that chemokine CCL11 at the N-terminal of the antigen protein can effectively induce the binding of antigen molecules and specific immune cells, thereby greatly enhancing the effect of crosspresentation of antigen molecules, so that CCL11 can finally induce a stronger specific immune response to antigen molecules. In addition, the T2 fragment added at the C-terminal of the antigenic protein can effectively enhance the immune strength against the antigen molecule in the process of cellular immune, which greatly increases the number of specific T cells, playing a decisive role as an immune enhancement factor.

### Example 5

The effects of chemokine CCL11 and T2 fragment on the humoral immune responses induced by fusion gene vaccine were explored. The vaccines comprising antigens of HPV16 E6 and E7 proteins were taken as an example, and the steps for inducing T cell responses by vaccines comprising other antigens were the same.

Whether CCL11 can also enhance humoral immune responses was next evaluated. Plasmids pVR-CCL11-E6E7-T2, pVR-CCL11-E6E7, and pVR-E6E7 were extracted separately, and the mice were immunized with the plasmids of 25 µg by an in vivo gene transfer instrument from TERESA. There were four groups including a negative control PBS group, 5 mice in each group. The mice were immunized according to the immunization strategy marked in the timeline in FIG. 4, and blood was collected from the mice in each group on D21 with no anticoagulant added. All the samples were centrifuged at 3000 rpm for 20 min. The supernatant was collected. On the first day, the E6E7 fusion protein purified in the laboratory was diluted with PBS, and coated onto an ELISA plate at 200 µL per well containing 10 µg of protein. The plate was then kept at 4°C overnight. On the second day, the plate was washed and blocked with blocking solution for 2 h at room temperature. The collected mouse serum was diluted by 1:100, and added into wells at 100 µL per well. The plate was then kept at 4°C overnight. On the third day, the color development was performed by ELISA. The results are shown in FIG. 6. The results show that in the comparison between groups at the same dose, the number of E7-specific antibodies in CCL11-E6E7-T2 group was far more than that in the E6E7 group. In the comparison between groups at the same dose, the number of E7-specific antibodies in CCL11-E6E7-T2 group was more than that in the CCL11-E6E7 group. In the comparison between groups at the same dose, the number of E7-specific antibodies in CCL11-E6E7 group was more than that in the E6E7 group. This shows that chemokine CCL11 at the N-terminal of the antigen protein can effectively induce the binding of antigen molecules and specific immune cells, thereby greatly enhancing the effect of crosspresentation of antigen molecules, so that CCL11 can finally induce a stronger specific immune response to antigen molecules. In addition, the T2 fragment added at the C-terminal of the antigenic protein can effectively enhance the immune strength against the antigen molecule in the process of cellular immune, which greatly increases the number of specific antibodies produced, playing a decisive role as an immune enhancement factor.

### Example 6

The therapeutic effects of fusion gene vaccines on mouse xenograft tumor cell TC-1 allograft tumor were explored. The fusion gene vaccines comprising antigens of HPV16 E6 and E7 proteins were taken as an example, and the steps for the demonstration of tumor intervention effect of vaccines comprising other antigens were the same.

In view of the excellent effects of vaccines such as CCL11-E6E7-T2 in the cellular and humoral immunity experiments, the therapeutic effects on the TC-1 allograft tumor after immunization with the fusion genes were further explored by experiments. In addition to the DNA vaccine form, the mRNA vaccine form of CCL11-E6E7-T2 was also synthesized, which was encapsulated with nanoparticles. The specific process is as follows: 5'UTR-CCL11-E6E7-3'UTR-A(120) was connected into the cloning vector pGEM-3Zf(+) (promega) to complete the construction of an *in vitro* transcription expression system, which was named pGEM -CCL 11- E6E7. The *in vitro* transcription system had a UTR sequence as follows:
5-UTR (β-globin-2) 
agagcggccgctttttcagcaagattaagcccagggcagagccatctattgcttacatttgcttctgacacaactgtgttcactagcaacctca
aacagacacc
3-UTR (2β-globin)

agctcgctttcttgctgtccaatttctattaaaggttcctttgttccctaagtccaactactaaactgggggatattatgaagggccttgagcatctg
gattctgcctaataaaaaacatttattttcattgcagctcgctttcttgctgtccaatttctattaaaggttcctttgttccctaagtccaactactaaac
tgggggatattatgaagggccttgagcatctggattctgcctaataaaaaacatttattttcattgc

The recombinant plasmid was linearized. The single enzyme digestion reaction system is shown in the table below, and the reaction was conducted at 37°C for 3 h.

| Component | Volume (µL) |
|---|---|
| Xho I | 4 |
| Recombinant plasmid | 12 µg |
| 10× CutSmart Buffer | 5 |
| dd H₂O | Balance |
| Total | 50 |

*In vitro* transcription reaction:
1. T7-Flash Scribe^{™} Transcription Kit (Cell script) was use for *in vitro* transcription, and UTP was replaced with N1-Methylpseudouridine-5'-Triphosphate (Trilink Biotech) during the preparation of *in vitro* transcription system.

The reaction system is shown in the table below. After the first step of reaction was completed, the reaction system was kept at 35°C for 30 min, and after the second step of reaction was completed, the reaction system was kept at 35°C for 15 min.

| Regents | Volume (µL) |
|---|---|
| Rnase-free Water | X |
| Linearized DNA | 1 µg |
| 10 ×T7 Transcription Solution | 2 |
| 100 mM ATP | 1.8 |
| 100 mM CTP | 1.8 |
| 100 mM GTP | 1.8 |
| 100 mM UTP | 1.8 |
| 100 mM DTT | 2 |
| Script Cuard Rnase Inhibitor | 0.5 |
| T7-Flash Scribe Enzyme Solution | 2 |
| Total | 20 |
| The above products | 20 |
| Rnase-free Dnase I | 1 |
| Total | 21 |

mRNA capping: The kit ScriptCap^{™} Cap 1 Capping System (Cell script) was used.

### Cap-mRNA purification

Purification was performed using the kit MEGAclear^{™} Kit Purification (Invitrogen).

### Preparation of liposome nanoparticles LNP-Man

DOTAP ((2,3-dioleoyl-propyl)trimethylammonium chloride), DOPE (dioleoylphosphatidylethanolamine), and DSPE-PEG2000 (distearoylphosphatidylethanolaminepolyethylene glycol 2000) were all purchased from Shanghai Advanced Vehicle Technology Co., Ltd.. DSPE-PEG2000-Man was purchased from Xi'an Haoran Biotechnology Co., Ltd.;
Nanoparticles were prepared by rotary evaporation method. The specific operation process of preparation of LNP-Man is as follows:
1) DOTAP, DOPE, and DSPE-PEG2000-Man were sequentially added to a round bottom flask according to the molar ratio of 50:50:1. 6 mL of chloroform was added until the solid was fully dissolved.
2) Ultrasonication in a water bath was performed for 15 min.
3) The round-bottom flask was put into a rotary evaporator, and the dissolved matter in the round-bottom flask should be submerged below the water surface. Then rotary evaporation was performed at a speed of 100 rpm for 15 min.
4) The round bottom flask was detached, placed in a fume hood, and added with 8 mL of HEPES buffer to dissolve the film on the inner wall of the bottle.
5) Ultrasonication in a water bath was performed for 30 min.
6) The ultrasonicated solution was filtered with a 0.22 µm filter membrane three times to obtain the desired liposome nanoparticles LNP-Man.

### Preparation of LNPs/mRNA

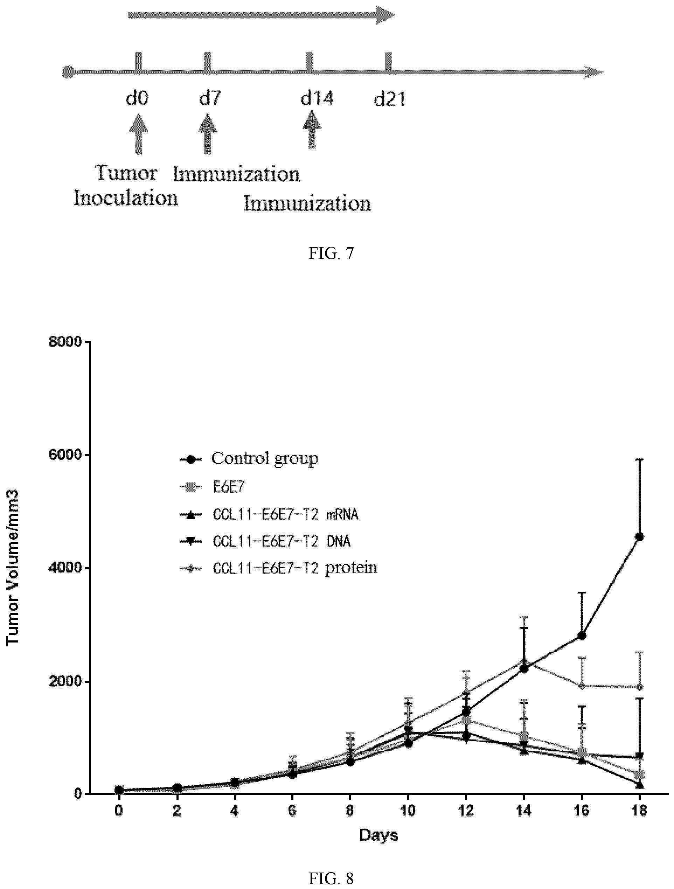

The prepared cationic lipid nanomaterial LNP-Man and mRNA were mixed according to the set N/P = 10:1 (molar ratio), and the required volume of LNPs and mRNA was calculated. LNPs and mRNA were respectively added with equal volumes of 10 mM HEPES buffer solution before mixing. The mixed LNPs/mRNA was shaken on a vortex shaker for 1 min, and left to stand at room temperature for 30 min. Moreover, CCL11-E6E7-T2 protein was also purified as a vaccine control. Mice were subjected to tumor inoculation and plasmid therapeutic immunization according to the timeline shown in FIG. 7, with an amount of each immunization of 25 µg. After tumor inoculation, the mice were immunized twice on D4 and D11, respectively. Afterwards, the tumors were measured in the same way, and the tumor volume was recorded. The tumor growth curve was drawn as shown in FIG. 8. The results show that the three groups pVR-CCL11-E6E7-T2 plasmid, CCL11-E6E7-T2-mRNA and CCL11-E6E7-T2 protein exhibited earlier tumor growth inhibition than the group pVR-E6E7 plasmid, and in the three groups pVR-CCL11-E6E7-T2 plasmid, CCL11-E6E7-T2-mRNA and CCL11-E6E7-T2 protein, the transplanted tumors completely disappeared around the 17th day after tumor inoculation, achieving a complete tumor treatment effect.

Finally, it should be stated that: the above embodiments are only intended for illustrating the technical solutions of the present disclosure rather than limiting the present disclosure. Although the present disclosure is illustrated in detail with reference to the embodiments described above, it should be understood by those skilled in the art that, modifications can still be made to the technical solutions recited in the embodiments described above, or equivalent substitutions can be made onto a part or all of the technical features of the technical solution. While such modifications or substitutions will not cause the essence of corresponding technical solutions to depart from the scope of the technical solutions of the embodiments of the present disclosure.

## Claims

1. Use of at least one of I)-VI) in improving antigen presentation effect, wherein
I) a T2 fragment with an amino acid sequence set forth in SEQ ID NO: 4;
II) chemokine CCL11;
III) a fragment being more than 80% homologous with and functionally identical or similar to I) or II);
IV) a nucleic acid molecule encoding I) or II);
V) a nucleic acid molecule that is derived from the nucleotide sequence of the nucleic acid molecule in IV) by deletion, addition or substitution of one or more nucleotides and encode a protein functionally identical or similar to the nucleic acid molecule in IV); and
VI) a nucleic acid molecule fully or partially complementary to V).

2. A fusion protein, comprising CCL11 and an antigen or comprising CCL11, an antigen and a T2 fragment.

3. The fusion protein according to claim 2, from N-terminal to C-terminal, sequentially comprising an IgE signal peptide, CCL11, a linker, the antigen and the T2 fragment.

4. The fusion protein according to claim 2 or 3, wherein the antigen is derived from a virus, pathogenic microbe and/or tumor;
the virus is selected from the group consisting of HPV, EBV, HCV, HIV, HBV, VZV, a coronavirus, and a combination thereof; and
the tumor is selected from the group consisting of liver cancer, cervical cancer, ovarian cancer, lung cancer, head and neck cancer, prostate cancer, breast cancer, blood cancer, ovarian cancer, colorectal cancer, and a combination thereof.

5. A nucleic acid molecule encoding the fusion protein according to any one of claims 2 to 4.

6. A nucleic acid fragment comprising the nucleic acid molecule according to claim 5, 5'-UTR, 3'-UTR and 3'-end polyA.

7. An expression vector comprising a vector backbone and the nucleic acid molecule according to claim 5 or the nucleic acid fragment according to claim 6.

8. A host transformed or transfected with the expression vector according to claim 7.

9. A method for producing the fusion protein according to any one of claims 2 to 4, comprising culturing the host according to claim 8 and collecting a culture containing the fusion protein.

10. Use of the fusion protein according to any one of claims 2 to 4, the nucleic acid molecule according to claim 5, the nucleic acid fragment according to claim 6, the expression vector according to claim 7, the host according to claim 8, the fusion protein prepared by the method according to claim 9, and/or the culture containing the fusion protein prepared by the method according to claim 9 in the manufacture of a product for preventing and/or treating a disease.

11. A product for preventing and/or treating a disease, comprising the fusion protein according to any one of claims 2 to 4, the nucleic acid molecule according to claim 5, the nucleic acid fragment according to claim 6, the expression vector according to claim 7, the host according to claim 8, the fusion protein prepared by the method according to claim 9, and/or the culture containing the fusion protein prepared by the method according to claim 9.

12. A method for preventing and/or treating a disease, comprising administering to a subject in need thereof the product according to claim 11.
